# FASCICULE DE BREVET EUROPEEN

(11) **EP 0 700 896 B1**
(45) Date de publication et mention de la délivrance du brevet: **06.11.1996**
(21) Numéro de dépôt: 95401800.8
(22) Date de dépôt: 31.07.1995
(51) Int. Cl.: C07C 229/16, A61K 7/42, A61K 7/48, C07D 215/26

(54) **Agents aptes à chélater les métaux, photoclivables, et compositions les contenant**
Photochemisch spaltbares, zur Chelation von Metallen fähiges Mittel und dieses enthaltende Zubereitungen
Photochemically cleavable agents able to chelate metals and compositions containing them

(30) Priorité: 08.09.1994 FR 9410763
(43) Date de publication de la demande: 13.03.1996
(73) Titulaire: L'OREAL, 75008 Paris (FR)
(72) Inventeur: Galey, Jean-Baptiste, F-93600 Aulnay-Sous-Bois (FR); Dumats, Jacqueline, F-93420 Villepinte (FR)
(74) Mandataire: Lhoste, Catherine

(56) Documents cités:
- EP-A- 0 313 305
- DE-A- 3 632 329
- FR-A- 2 698 095
- CHEMICAL ABSTRACTS, vol. 97, no. 3, 19 Juillet 1982, Columbus, Ohio, US; abstract no. 018948, S. VAVERKOVA et al, "Quinoline derivatives as wild chamomile growth biostimulators"

## Description

La présente invention a pour objet un nouvel agent apte à chélater un métal de transition et les compositions, notamment cosmétiques et/ou dermatologiques et/ou de l'hygiène, contenant au moins un tel agent apte à chélater. Ces compositions sont destinées en particulier à protéger la peau, les cheveux et/ou les muqueuses contre les effets induits par le rayonnement lumineux, notamment ultraviolet, et à prévenir et/ou lutter contre le photovieillissement cutané. Il est connu que les espèces activées de l'oxygène (oxygène singulet, anion superoxyde, eau oxygénée et radical hydroxyle) participent au processus de photovieillissement et les travaux sur le sujet sont nombreux (voir par exemple P. Morlière et al., Path. Biol., 1992, vol. 40 (2), p. 160-168). On sait aussi que les métaux de transition, et en particulier le fer et le cuivre, jouent un rôle essentiel dans les réactions moléculaires mises en jeu au niveau des tissus lors de leur exposition au rayonnement lumineux. Ces réactions créent des dommages appelés "dommages oxydatifs" qui entraînent un vieillissement prématuré de la peau (voir par exemple C.W. Trenam et al., J. Invest. Dermatol., 1992, vol. 99, p.675-682, et, T.P. Ryan et al., Critical Reviews in Toxicology, 1992, vol. 22 (1), p.119-141).

Il est connu que les espèces activées de l'oxygène (oxygène singulet, anion superoxyde, eau oxygénée et radical hydroxyle) participent au processus de photovieillissement et les travaux sur le sujet sont nombreux (voir par exemple P. Morlière et al., Path. Biol., 1992, vol. 40 (2), p. 160-168). On sait aussi que les métaux de transition, et en particulier le fer et le cuivre, jouent un rôle essentiel dans les réactions moléculaires mises en jeu au niveau des tissus lors de leur exposition au rayonnement lumineux. Ces réactions créent des dommages appelés "dommages oxydatifs" qui entraînent un vieillissement prématuré de la peau (voir par exemple C.W. Trenam et al., J. Invest. Dermatol., 1992, vol. 99, p.675-682, et, T.P. Ryan et al., Critical Reviews in Toxicology, 1992, vol. 22 (1), p.119-141).

Par ailleurs, le fer et, dans une moindre mesure, le cuivre sont des éléments essentiels à la vie et ils jouent un rôle déterminant dans de nombreux phénomènes biologiques. Ces éléments sont présents en plusieurs endroits dans les tissus vivants, et principalement au sein des sites actifs des métalloenzymes, ou bien stockés dans des protéines de réserve ou de transport. Le fer intervient notamment pour le transport, le stockage et l'activation de l'oxygène (voir M. Fontecave et al., Bull. Soc. Chim., 1993, vol. 130, p.77-85).

En situation normale, ces métaux, et en particulier le fer, ne sont jamais sous forme libre ni accessible, et ne peuvent donc pas catalyser les échanges redox conduisant à des dommages oxydatifs. En revanche, en situation de "stress oxydant", en particulier lors d'exposition au rayonnement lumineux, de faibles quantités de métaux peuvent être libérées et devenir ainsi disponibles pour catalyser ces réactions. Il a été notamment observé que la ferritine libérait du fer sous l'action des rayons ultraviolets (voir M. Aubailly et al., Photochem. Photobiol., 1991, vol. 54 (5), p.769-773).

Pour éviter ce phénomène, il est connu d'utiliser des chélateurs de métaux de transition, et notamment des chélateurs de fer, qui, en captant le fer, inhibent les processus oxydatifs qu'il pourrait provoquer.

Ainsi, les documents EP-A-313305, EP-A-496433 et EP-A-496434 décrivent des compositions photoprotectrices utilisées en application topique et comprenant des agents chélateurs qui captent le fer libre et qui sont destinés à prévenir les dommages sur la peau provoqués par l'exposition aux rayons ultraviolets.

Néanmoins, ces agents chélateurs présentent des effets secondaires, et notamment des risques de toxicité chronique, et ces risques limitent leur utilisation comme agents de protection de la peau. En effet, ces agents chélateurs peuvent interférer avec le métabolisme du fer qui, du fait de son rôle vital dans le fonctionnement des cellules du corps humain, doit être préservé, et ils peuvent notamment bloquer le fer, même celui nécessaire au fonctionnement des tissus. On cherche par conséquent à éviter ou du moins à limiter l'utilisation de ces agents chélateurs pour toute substance devant être en contact avec le tissu vivant.

Il subsiste donc le besoin d'agents chélateurs des métaux de transition, qui empêchent les dégâts causés par ces métaux, et notamment le fer et le cuivre, lors de l'exposition au rayonnement lumineux, et notamment ultraviolet, sans présenter les inconvénients des agents chélateurs habituellement utilisés pour capter ces métaux.

Par ailleurs, on connaît des dérivés nitrés de la quinoléine utilisés comme intermédiaires de synthèse (voir document DE-A-3632329) et comme biostimulateurs de la croissance de la camomille (voir Chemical Abstract, vol.097, n°3, 018948).

Il a été maintenant trouvé de manière surprenante que ces composés et d'autres peuvent être utilisés comme agents aptes à chélater les métaux de transition. Les agents selon l'invention présentent la particularité de n'acquérir leur propriété chélatrice que lors d'exposition au rayonnement lumineux, c'est-à-dire seulement lorsqu'il est nécessaire de neutraliser les traces des métaux intervenant dans les phénomènes de dommages oxydatifs. Les agents de l'invention permettent donc de limiter les effets secondaires des agents chélateurs de l'état de la technique.

La présente invention se rapporte donc à l'utilisation comme agent apte à chélater un métal de transition suite à une exposition au rayonnement lumineux, d'un composé comportant un groupement comportant au moins une fonction chélatrice d'un métal de transition, bloquée par au moins un substituant photoclivable.

Les métaux de transition auxquels s'applique l'invention sont les métaux ayant un numéro atomique compris dans la gamme allant de 21 à 30, et il s'agit plus particulièrement du fer et du cuivre.

Les agents aptes à chélater les métaux de transition selon l'invention possèdent au moins une fonction chélatrice bloquée par un substituant qui a la particularité de se séparer de la ou des fonctions chélatrices lors d'une exposition au rayonnement lumineux et de libérer alors la ou les fonctions chélatrices qui vont pouvoir jouer leur rôle chélateur. De ce fait, lorsqu'ils ne sont pas exposés au rayonnement lumineux, les agents de l'invention ont une affinité très faible, voire nulle, pour le fer ou le cuivre, et ne peuvent donc avoir d'effets secondaires néfastes sur le métabolisme du fer et/ou du cuivre. Par contre, lorsqu'ils sont exposés au rayonnement lumineux, ils libèrent la ou les fonctions chélatrices qui ont une forte affinité pour les métaux de transition et vont capter le métal libéré, empêchant ainsi la dégradation des tissus.

L'invention a aussi pour objet une composition comprenant un agent apte à chélater un métal de transition suite à une exposition au rayonnement lumineux, caractérisée par le fait que le dit agent comporte un groupement comportant au moins une fonction chélatrice d'un métal de transition, bloquée par un substituant photoclivable et en ce qu'elle comprend en outre un milieu cosmétiquement et/ou dermatologiquement acceptable.

L'invention a encore pour objet une composition pour protéger la peau, les cheveux, et plus spécialement le cuir chevelu, et/ou les muqueuses, contre les effets induits par le rayonnement lumineux et pour prévenir et/ou lutter contre le photovieillissement cutané, caractérisée en ce qu'elle consiste en une composition telle que définie ci-dessus.

L'invention a aussi pour objet l'utilisation d'un agent apte à chélater un métal de transition suite à une exposition au rayonnement lumineux, le dit agent comportant un groupement comportant au moins une fonction chélatrice bloquée par au moins un substituant photoclivable dans une composition cosmétique et/ou dermatologique pour protéger la peau, les cheveux et/ou les muqueuses contre les effets induits par le rayonnement lumineux et/ou pour prévenir et/ou lutter contre le photovieillissement cutané.

L'invention a en outre pour objet l'utilisation de la composition telle que définie ci-dessus pour préparer une pommade et/ou un onguent dermatologiques destinés à protéger la peau, les cheveux et/ou les muqueuses contre les effets induits par le rayonnement lumineux et/ou à prévenir et/ou lutter contre le photovieillissement cutané.

L'invention a enfin pour objet un procédé de traitement cosmétique de la peau, des cheveux et/ou des muqueuses consistant à appliquer sur la peau, les cheveux et/ou les muqueuses, la composition telle que définie ci-dessus.

La fonction chélatrice de l'agent selon l'invention peut être tout fonction chélatrice des métaux de transition, et notamment une fonction amine, carbonyle, nitrile, oxime, carboxylique, hydroxyle, hydroxamique, alcoxyde, énolique, phénolique, phénoxyde, hydrazide, soufrée et leurs associations.

On peut citer par exemple comme groupement chélateur comportant une ou plusieurs de ces fonctions les amines aromatiques ou aliphatiques, les cétones aliphatiques ou aromatiques, les aldéhydes aliphatiques ou aromatiques, les dioximes et cétoximes, les acides carboxyliques aliphatiques ou aromatiques ainsi que leurs esters et leurs sels, les phénols et leurs dérivés, les hydroxy-acides carboxyliques aliphatiques ou aromatiques, les hydrazides, les catécholates, les cétoénolates, les hydroxamates, les hydroxy-amines aromatiques.

On peut citer en particulier comme groupement chélateur comportant plusieurs de ces fonctions les hydroxypyridinones, les amines dicarboxyliques, les o-hydroxybenzylamines, les hydroxamates.

On peut citer plus spécialement comme groupement chélateur :
- les hydroxypyridinones de structure : 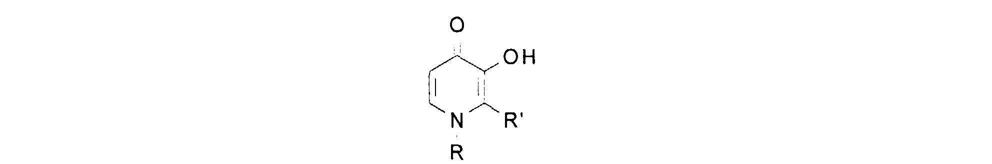 où R et R' représentent indépendamment l'un de l'autre un reste alkyle, notamment méthyle.
- les dérivés aminocarboxylates de structure : 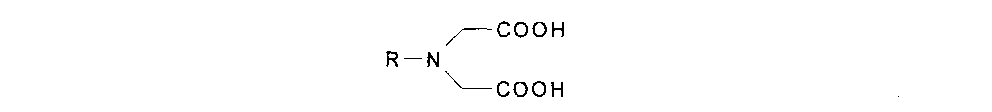 où R représente un reste alkyle, éventuellement porteur d'autres fonctions aminocarboxylates. Il peut s'agir par exemple de l'acide diéthylènetriamine-pentaacétique (DTPA).
- les dérivés o-hydroxybenzylamines de structure : 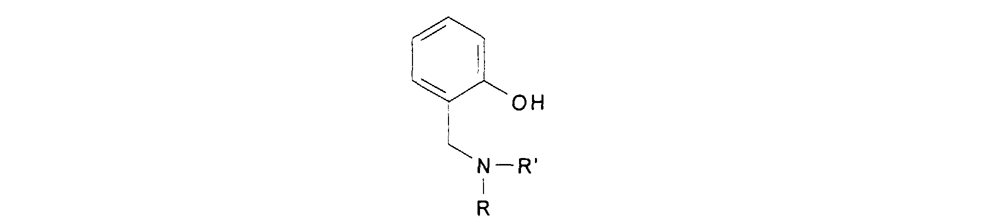 où R et R' représentent indépendamment l'un de l'autre un reste acétique ou alkyle éventuellement porteur d'autres fonctions chélatrices. Il peut s'agir par exemple de l'acide N,N'-bis--(2-hydroxybenzyl)-éthylènediaminediacétique.
- les dérivés hydroxamates de structure : 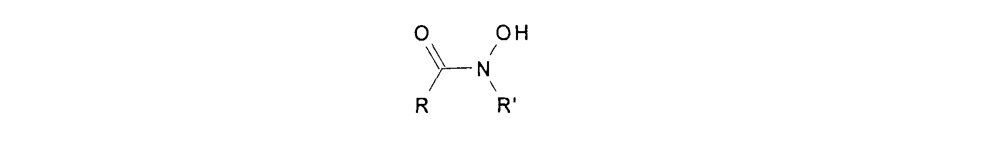 où R et R' représentent indépendamment l'un de l'autre une chaîne aminoacide, portant éventuellement d'autres fonctions hydroxamate. Il peut s'agir par exemple de la desferioxamine B.

Le blocage des fonctions chélatrices peut être réalisé avec les groupes protecteurs photoclivables classiquement utilisés en chimie de synthèse, ces groupes variant selon la nature de la fonction chélatrice à bloquer.

Il s'agit notamment de groupes p-méthoxyphénacyle, 2-nitrobenzyle, 2-nitrobenzyloxycarbonyle, 2-nitrophényléthylèneglycol, benzyloxycarbonyle, 3,5-diméthoxybenzyloxycarbonyle, α,α-diméthyl-3,5-diméthoxybenzyloxycarbonyle, 3-nitrophényle, 3-nitrophényloxy, 3,5-dinitrophényloxy, 3-nitrophényloxycarbonyle, phénacyle, 4-méthoxyphénacyle, α-méthylphénacyle, 3,5-diméthoxybenzoïnyle, 2,4-dinitrobenzènesulfényle.

L'agent apte à chélater un métal de transition selon l'invention peut être par exemple un composé qui, par photoclivage, va donner lieu à la formation d'amines aromatiques, d'amines, de phénols, de composés carbonylés ou d'alcools.

Comme composés donnant par photoclivage une amine aromatique, on peut citer par exemple les amines aromatiques N-oxydes.

Comme composés donnant par photoclivage une amine, on peut citer par exemple les formamides d'aryle, les carbamates de benzyle, les benzylsulfonamides, les toluènesulfonamides, les carbamates de 3,5-diméthoxybenzyle, les carbamates de 3,4-diméthoxy-6-nitrobenzyle, les carbamates de 1-méthyl-1-(3,5-diméthoxyphényl)-éthyle, les N-o-nitrobenzylamines, les carbamates de o-nitrobenzyle ou les carbamates de m-nitrophenyle.

Comme composés donnant par photoclivage un acide carboxylique, on peut citer par exemple les esters bis(o-nitrophényl)méthyliques, les esters α-(3,5-diméthoxyphényl)phénacyliques, les esters 2,4-dinitrophénylsulfényliques, les esters 2-(9,10-dioxo)anthrylméthyliques, les esters p-méthoxyphénacyliques, les esters α-méthylphénacyliques, les nitroamides, les o-nitroanilides, les esters o-nitrobenzyliques, les N-7-nitroindolylamides, les N-8-nitro-1,2,3,4-tetrahydroquinolylamides.

Comme composés donnant par photoclivage un phénol, on peut citer par exemple les esters 9-fluorènecarboxyliques, les éthers d'o-nitrobenzyle, les esters xanthènecarboxyliques.

Comme composés donnant par photoclivage un composé carbonylé, on peut citer par exemple les S,S-dibenzylacétals, les N,N-diméthylhydrazones, les 1,3-dithiolanes, les 1,3-oxathiolanes.

Comme composés donnant par photoclivage un alcool, on peut citer par exemple les nitrates, les carbonates d'o-nitrobenzyle, les éthers d'o-nitrobenzyle.

Les groupes protecteurs photoclivables utilisés de préférence dans l'invention sont en particulier:
- les esters p-méthoxyphénacyliques qui protègent les fonctions chélatrices carboxyliques et dont la réaction de photoclivage sous U.V. est la suivante: 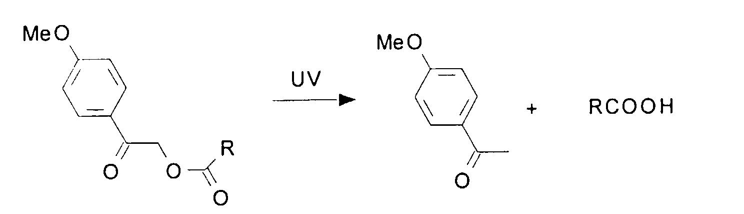 où RCOOH représente le composé chélateur ne comportant plus de groupement protecteur, et donc capable de capter les métaux de transition.
- les éthers o-nitrobenzyliques qui protègent les fonctions chélatrices phénols, dont la réaction de photoclivage sous U.V. est la suivante : 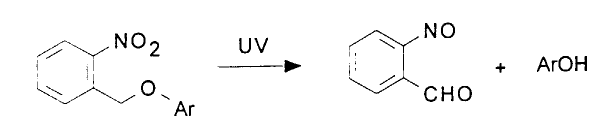 où ArOH représente le composé chélateur ne comportant plus de groupement protecteur, et donc capable de capter les métaux de transition.

Plus particulièrement, les agents chélateurs photoactivables préférés de l'invention ont les formules suivantes : 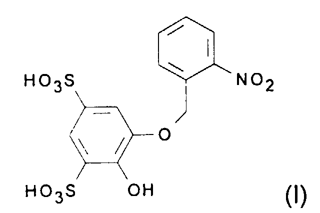 Acide 2-(o-nitrobenzyloxy)- phénol-4,6-disulfonique 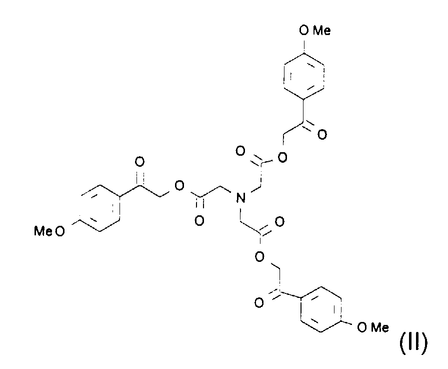 Triester p-méthoxyphénacylique d'acide nitrilotriacétique 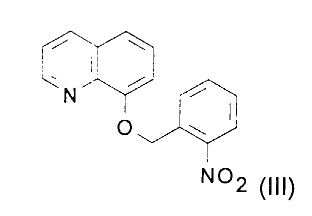 8-(2-nitrobenzyloxy)-quinoléine 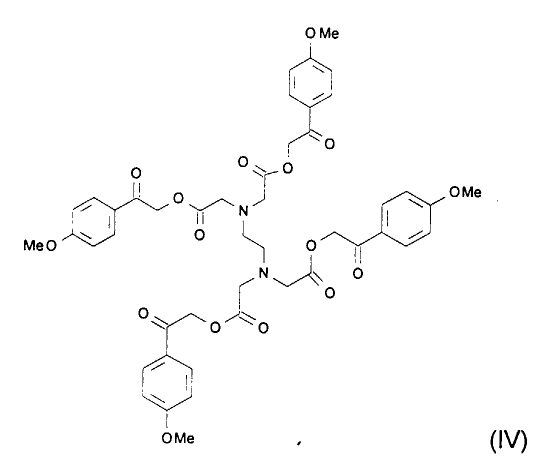 Tétraester p-méthoxyphénacylique d'acide éthylènediamine tétracétique

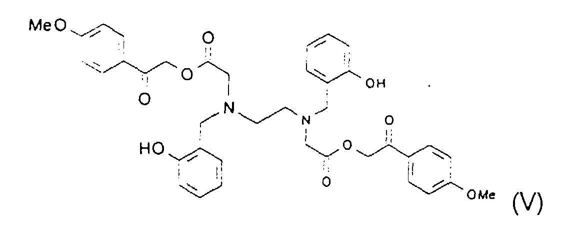

Diester p-méthoxyphénacylique d'acide bis-N,N'-(2-hydroxybenzyl)éthylènediamine diacétique 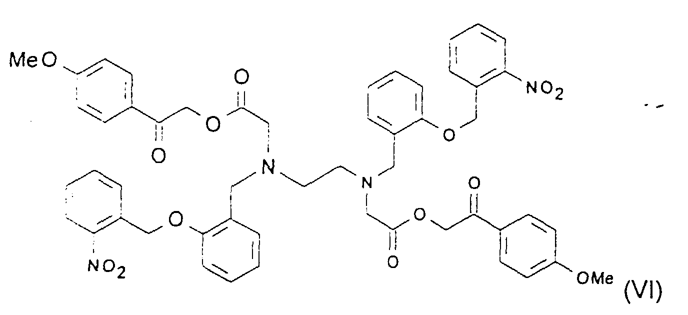 Diester p-méthoxyphénacylique d'acide bis-N,N'-2-(o-nitrobenzyloxybenzyl)éthylènediamine diacétique

L'agent chélateur selon l'invention peut avantageusement être utilisé dans une composition cosmétique et/ou dermatologique en une concentration allant de 0,001 % à 10 % en poids, et de préférence de 0,05 % à 5 % en poids par rapport au poids total de la composition.

Les compositions selon l'invention comportent un milieu cosmétiquement et/ou dermatologiquement acceptable, c'est-à-dire compatible avec la peau. Elles peuvent se présenter sous toutes les formes galéniques normalement utilisées pour une application topique, destinées particulièrement aux domaines cosmétique et/ou dermatologique. En particulier, les compositions peuvent se présenter sous forme de solutions aqueuses, alcooliques ou hydroalcooliques, sous forme de crèmes, de gels hydrophiles ou lipophiles, d'émulsions eau-dans-huile ou huile-dans-eau, de crèmes ou de gels aptes à mousser, de compositions en aérosol, ou encore sous forme de microgranulés, de poudres ou de dispersions vésiculaires de type ionique et/ou non ionique. Ces compositions sont préparées selon les méthodes usuelles des domaines considérés.

Les quantités des différents constituants des compositions selon l'invention sont celles classiquement utilisées dans les domaines cosmétique ou dermatologique ou de l'hygiène.

Ces compositions constituent notamment des compositions de nettoyage, de protection, de traitement ou de soin pour le visage, pour le cou, pour les mains ou pour le corps (par exemple crèmes de jour, crèmes démaquillantes, crèmes ou huiles solaires, laits de nettoyage, laits de démaquillage, des laits corporels), des compositions de maquillage (par exemple fonds de teint), des compositions de bronzage artificiel.

Elles peuvent être également utilisées dans diverses compositions pour les cheveux, et notamment des lotions traitantes, des crèmes ou des gels coiffants, des compositions de teintures, des lotions ou des gels antichute.

Lorsque la composition de l'invention est une émulsion, la proportion de corps gras peut aller de 5 % à 80 % en poids, et de préférence de 5 % à 50 % en poids par rapport au poids total de la composition. Les corps gras et les émulsionnants utilisés dans la composition sous forme d'émulsion sont choisis parmi ceux classiquement utilisés dans le domaine cosmétique ou dermatologique. Les émulsionnants peuvent être présents, dans la composition, en une proportion allant de 0,3 % à 30 % en poids, et de préférence de 0,5 à 30 % en poids par rapport au poids total de la composition. L'émulsion peut, en outre, contenir des vésicules lipidiques.

De façon connue, la composition cosmétique ou dermatologique de l'invention peut contenir également des adjuvants habituels dans le domaine cosmétique ou dermatologique, tels que les gélifiants hydrophiles ou lipophiles, les actifs hydrophiles ou lipophiles, les conservateurs, les antioxydants, les solvants (alcools), les parfums, les charges, les filtres et les matières colorantes. Les quantités de ces différents adjuvants sont celles classiquement utilisées dans les domaines considérés, et par exemple de 0,01 % à 20 % en poids par rapport au poids total de la composition. Ces adjuvants, selon leur nature, peuvent être introduits dans la phase grasse, dans la phase aqueuse et/ou dans les vésicules lipidiques.

Comme corps gras utilisables dans l'invention, on peut citer les huiles minérales, les huiles végétales (huile de jojoba) et leurs dérivés hydrogénés , les huiles animales (lanoline), les huiles de synthèse (myristate d'isopropyle), les huiles siliconées (cyclopentadiméthylsiloxane) et les huiles fluorées. On peut ajouter à ces huiles d'autres corps gras tels que les alcools gras (alcool cétylique, alcool stéarylique), les acides gras (acide stéarique) et les cires.

Comme gélifiants hydrophiles, on peut citer par exemple les polymères carboxyvinyliques (carbomer), les copolymères acryliques, les polyacrylamides, les polysaccharides, les gommes naturelles et les argiles, et, comme gélifiants lipophiles, on peut citer les argiles modifiées et les sels métalliques d'acides gras.

Comme actifs, on peut citer par exemple les protéines ou les hydrolysats de protéine, les acides aminés, les polyols, les sucres et les dérivés de sucre, les vitamines, les hydroxyacides, les céramides, les huiles essentielles, l'acide salicylique et ses dérivés.

Les exemples qui suivent illustrent les procédés de préparation d'agents conformes à l'invention.

### Exemple 1 : préparation du triester p-méthoxyphénacylique d'acide nitrilotriacétique (composé de formule II)

On solubilise 1 g d'acide nitrilotriacétique dans 15 ml de diméthylformamide contenant 2,3 ml de triéthylamine. On refroidit le mélange à 0°C, puis on y ajoute 3,6 g de 2-bromo-4'-méthoxyacétophénone en solution dans 20 ml de diméthylformamide (DMF). On maintient le mélange pendant 24 heures à 0°C. On verse ensuite le mélange sur 150 g de glace et on fait une extraction par trois fois dans 50 ml de dichlorométhane. La phase organique est lavée à l'eau, séchée sur du sulfate de sodium, puis évaporée à sec. Le produit huileux obtenu est purifié par passage sur colonne de silice avec, comme éluant, le mélange dichlorométhane/méthanol (95:5).

On obtient 0,8 g de triester p-méthoxyphénacylique d'acide nitrilotriacétique sous forme d'huile (rendement non optimisé : 25%).

Le spectre RMN ¹H 500MHz dans le diméthylsulfoxyde (DMSOd⁶) est en accord avec la structure :

**RMN** ^{**1**}**H 500 MHz** (DMSOd⁶, δ ppm) : 3,86 (9H, s) ; 3,88 (6H, s) ; 5,51 (6H, s) 7,08 (6H, dd) ; 7,96 (6H, dd).

### Exemple 2 : préparation du 8-(2-nitrobenzyloxy)-quinoléine (composé de formule III)

On solubilise 1,5 g de 8-hydroxyquinoléine dans 30 ml de méthanol sec contenant 0,7 g de méthylate de sodium. Après 15 minutes à température ambiante, la solution est évaporée à sec. Le produit obtenu est repris dans 5 ml de diméthylformamide et à nouveau évaporé. Puis on le dissout dans 30 ml de DMF et on y additionne 2,3 g de chlorure de 2-nitrobenzyle en solution dans 15 ml de DMF. On agite la solution pendant 45 minutes à la température ambiante. Puis, le mélange est alors évaporé à sec et le résidu obtenu est dissous dans de l'acétate d'éthyle. Ensuite il est lavé à l'eau, séché sur du sulfate de sodium et évaporé à sec. Après extraction par trois fois dans 50 ml d'éther de pétrole, l'insoluble obtenu est recristallisé dans 50 ml d'éthanol.

On obtient 1,2 g de 8-(2-nitrobenzyloxy)-quinoléine sous forme de produit solide jaune (rendement non optimisé 40%).

Point de fusion : 152°C.

Le spectre RMN ¹H dans le chloroforme deutérié (CDCl₃) est conforme à la structure :

**RMN** ^{**1**}**H 500 MHz** (CDCl₃, δ ppm) : 5,83 (2H, s) ; 7,00 (1H, dd) ; 7,40 (4H, m) 7,63 (1H, td) ; 8,06 (1H, dd) ; 8,13 (1h, dd) ; 8,17 (1H, dd) ; 8,98 (1H, dd).

### Exemple 3 : préparation du diester p-méthoxyphénacylique de l'acide bis-N,N'-(2-hydroxybenzyl)-éthylènediaminediacétique (Composé de formule V)

On solubilise 1 g d'acide bis-N,N'-(2-hydroxybenzyl)-éthylènediaminediacétique dans 10 ml de diméthylformamide contenant 1,2 ml de triéthylamine. On refroidit le mélange à 0°C, puis on y ajoute 1 g de 2-bromo-4'-méthoxyacétophénone en solution dans 10 ml de diméthylformamide. On maintient le mélange pendant 24 heures à 0°C. On verse ensuite le mélange sur 100 g de glace et on fait une extraction par trois fois dans 50 ml de dichlorométhane. La phase organique est lavée à l'eau, séchée sur du sulfate de sodium, puis évaporée à sec. L'huile obtenue est solubilisée dans le minimum de dichlorométhane et on y ajoute de l'isopropanol jusqu'à précipitation du milieu. Le précipité est filtré, puis repris par deux fois par de l'éthanol au reflux.

On obtient 200 mg de diester p-méthoxyphénacylique de l'acide bis-N,N'-2-hydroxybenzyléthylènediaminediacétique sous forme d'un produit blanc (rendement non optimisé : 15 %)

Point de fusion : 178°C.

Le spectre RMN ¹H 500MHz dans le DMSO est en accord avec la structure :

**RMN** ^{**1**}**H 500 MHz** (DMSOd⁶, δ ppm) : 2,80 (4H, s) ; 3,56 (4H, s) ; 3,77 (4H, s) 3,86 (6H, s) ; 5,47 (4H, s) ; 6,75 (4H, m) ; 7,07 (4H, dd) ; 7,11 (4H, dd) ; 7,94 (4H, dd) ; 9,62 (2H, s).

Les exemples suivants illustrent les compositions cosmétiques ou dermatologiques selon l'invention. Les quantités sont données en pourcentage en poids.

### Exemple 1 : crème solaire

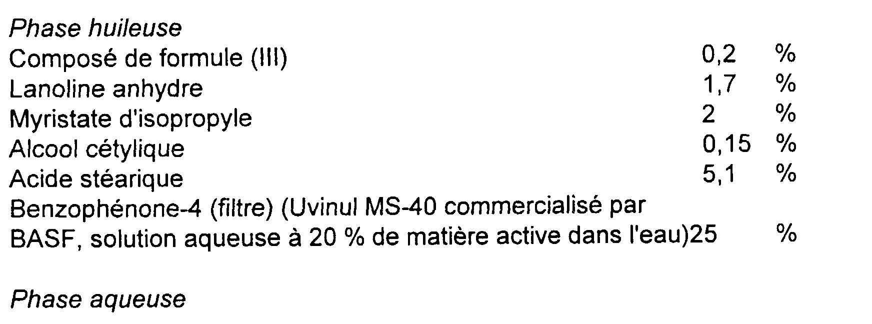

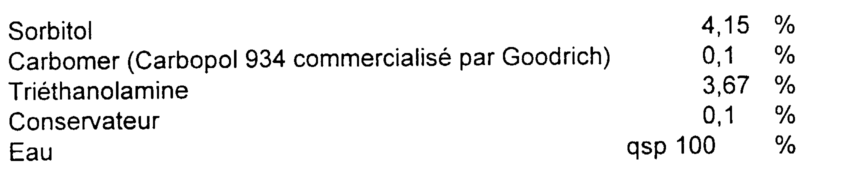

Le mode opératoire consiste à mélanger l'eau, le carbomer et le sorbitol, puis à ajouter une partie de la triéthanolamine, à préparer par ailleurs la phase huileuse par mélange à chaud (environ 75°C) des différents constituants sauf le filtre, puis à introduire la phase huileuse dans la phase aqueuse sous agitation, et à introduire ensuite le filtre après avoir ajusté le pH de la solution le contenant avec le reste de triéthanolamine.

On obtient une crème solaire de couleur blanc cassé, qui assure une bonne protection contre les effets néfastes des rayons ultraviolets.

### Exemple 2 : Crème de soin hydratante

| | |
|---|---|
| *Phase huileuse* | |
| Composé de formule (VI) | 0,1 % |
| Huile de jojoba | 13 % |
| Alcool stéarylique | 1 % |
| Acide stéarique | 4 % |
| Cyclopentadiméthylsiloxane | 10 % |
| Vitamine E | 1 % |
| Stéarate de polyéthylèneglycol | 3 % |
| | |
| *Phase aqueuse* | |
| Sorbate de potassium | 0,3 % |
| Glycérol | 3 % |
| Conservateur | 0.05 % |
| Eau | qsp 100 % |

Le mode opératoire consiste à mélanger d'une part les constituants de la phase aqueuse, d'autre part les constituants de la phase huileuse, et à introduire ensuite sous agitation la phase huileuse dans la phase aqueuse.

On obtient une crème blanche qui hydrate bien la peau et la protège du rayonnement lumineux.

## Revendications

1. Utilisation comme agent apte à chélater un métal de transition suite à une exposition au rayonnement lumineux, d'un composé comportant un groupement comportant au moins une fonction chélatrice d'un métal de transition, bloquée par au moins un substituant photoclivable.

2. Utilisation selon la revendication 1, caractérisé en ce que la fonction chélatrice de l'agent chélateur est choisie parmi les fonctions amine, carbonyle, nitrile, oxime, carboxylique, hydroxyle, hydroxamique, alcoxyde, énolique, phénolique, phénoxyde, hydrazide, soufrée et leurs associations.

3. Utilisation selon la revendication 1 ou 2, caractérisé en ce que le groupement de l'agent chélateur est choisi parmi les hydroxypyridinones, les amines dicarboxyliques, les o-hydroxybenzylamines, les hydroxamates.

4. Utilisation selon l'une quelconque des revendications 1 à 3, caractérisé en ce que le substituant photoclivable de l'agent chélateur est choisi parmi les groupes p-méthoxyphénacyle, 2-nitrobenzyle, 2-nitrobenzyloxycarbonyle, 2-nitrophényléthyléneglycol, benzyloxycarbonyle, 3,5-diméthoxybenzyloxy-carbonyle, α,α-diméthyl-3,5-diméthoxybenzyloxycarbonyle, 3-nitrophényle, 3-nitrophényloxy, 3,5-dinitrophényloxy, 3-nitrophényloxycarbonyle, phénacyle, 4-méthoxyphénacyle, α-méthylphénacyle, 3,5-d iméthoxybenzoïnyle, 2,4-dinitro-benzènesulfényle.

5. Utilisation selon l'une quelconque des revendications 1 à 4, caractérisé en ce que le substituant photoclivable de l'agent chélateur est choisi parmi les groupes p-méthoxyphénacyle et o-nitrobenzyle.

6. Utilisation selon l'une quelconque des revendications 1 à 5, caractérisé en ce que le métal de transition a un numéro atomique choisi dans la gamme allant de 21 à 30.

7. Utilisation selon l'une quelconque des revendications 1 à 6, caractérisé en ce que le métal de transition est choisi dans le groupe formé par le fer et le cuivre.

8. Composition comprenant un agent apte à chélater un métal de transition suite à une exposition au rayonnement lumineux, caractérisée par le fait que le ditagent comporte un groupement comportant au moins une fonction chélatrice d'un métal de transition, bloquée par au moins un substituant photoclivable et en ce qu'elle comprend en outre un milieu cosmétiquement et/ou dermatologiquement acceptable.

9. Composition selon la revendication 8, caractérisée par le fait que la fonction chélatrice est choisie parmi les fonctions amine, carbonyle, nitrile, oxime, carboxylique, hydroxyle, hydroxamique, alcoxyde, énolique, phénolique, phénoxyde, hydrazide, soufrée et leurs associations.

10. Composition selon la revendication 8 ou 9, caractérisée par le fait que le groupement de l'agent chélateur est choisi parmi les hydroxypyridinones, les amines dicarboxyliques, les o-hydroxybenzylamines, les hydroxamates.

11. Composition selon l'une quelconque des revendications 8 à 10, caractérisée par le fait que le substituant photoclivable est choisi parmi les groupes p-méthoxyphénacyle, 2-nitrobenzyle, 2-nitrobenzyloxycarbonyle, 2-nitrophényléthylèneglycol, benzyloxycarbonyle, 3,5-diméthoxybenzyloxycarbonyle, α,α-diméthyl-3,5-diméthoxybenzyloxycarbonyle, 3-nitrophényle, 3-nitrophényloxy, 3,5-dinitrophényloxy, 3-nitrophényloxycarbonyle, phénacyle, 4-méthoxyphénacyle, α-méthylphénacyle, 3,5-diméthoxybenzoïnyle, 2,4-dinitrobenzéne-sulfényle.

12. Composition selon l'une quelconque des revendications 8 à 11, caractérisée par le fait que le substituant photoclivable est choisi parmi les groupes p-méthoxyphénacyle et o-nitrobenzyle.

13. Composition selon l'une quelconque des revendications 8 à 12, caractérisée par le fait que le métal de transition a un numéro atomique choisi dans la gamme allant de 21 à 30.

14. Composition selon l'une quelconque des revendications 8 à 13, caractérisée par le fait que le métal de transition est choisi dans le groupe formé par le fer et le cuivre.

15. Composition pour protéger la peau, les cheveux et/ou les muqueuses contre les effets induits par le rayonnement lumineux et/ou pour prévenir et/ou lutter contre le photovieillissement cutané, caractérisée en ce qu'elle consiste en une composition selon l'une quelconque des revendications 8 à 14.

16. Utilisation d'un agent apte à chélater un métal de transition suite à une exposition au rayonnement lumineux, le dit agent comportant un groupement comportant au moins une fonction chélatrice bloquée par au moins un substituant photoclivable dans une composition cosmétique et/ou dermatologique pour protéger la peau, les cheveux et/ou les muqueuses contre les effets induits par le rayonnement lumineux et/ou pour prévenir et/ou lutter contre le photovieillissement cutané.

17. Utilisation de la composition selon l'une quelconque des revendications 8 à 14 pour préparer une pommade et/ou un onguent dermatologiques destinés à protéger la peau, les cheveux et/ou les muqueuses contre les effets induits par le rayonnement lumineux et/ou à prévenir et/ou lutter contre le photovieillissement cutané.

18. Procédé de traitement cosmétique de la peau, des cheveux et/ou des muqueuses consistant à appliquer sur la peau, les cheveux et/ou les muqueuses, la composition selon l'une quelconque des revendications 8 à 14.

## Claims

1. Use, as an agent capable of chelating a transition metal following an exposure to luminous radiation, of a compound containing a group containing at least one chelating functional group for a transition metal, blocked by at least one photocleavable substituent.

2. Use according to Claim 1, characterized in that the chelating functional group of the chelating agent is chosen from amine, carbonyl, nitrile, oxime, carboxylic, hydroxyl, hydroxamic, alkoxy, enolic, phenolic, phenoxy, hydrazide and sulphur-containing functional groups and combinations thereof.

3. Use according to Claim 1 or 2, characterized in that the group of the chelating agent is chosen from hydroxypyridinones, dicarboxylic amines, o-hydroxybenzylamines and hydroxamates.

4. Use according to any one of Claims 1 to 3, characterized in that the photocleavable substituent of the chelating agent is chosen from p-methoxyphenacyl, 2-nitrobenzyl, 2-nitrobenzyloxycarbonyl, 2-nitrophenylethylene glycol, benzyloxycarbonyl, 3,5-dimethoxybenzyloxycarbonyl, α,α-dimethyl-3,5-dimethoxybenzyloxycarbonyl, 3-nitrophenyl, 3-nitrophenoxy, 3,5-dinitrophenoxy, 3-nitrophenoxycarbonyl, phenacyl, 4-methoxyphenacyl, α-methylphenacyl, 3,5-dimethoxybenzoinyl and 2,4-dinitrobenzenesulphenyl groups.

5. Use according to any one of Claims 1 to 4, characterized in that the photocleavable substituent of the chelating agent is chosen from p-methoxyphenacyl and o-nitrobenzyl groups.

6. Use according to any one of Claims 1 to 5, characterized in that the transition metal has an atomic number chosen from the range running from 21 to 30.

7. Use according to any one of Claims 1 to 6, characterized in that the transition metal is chosen from the group consisting of iron and copper.

8. Composition including an agent capable of chelating a transition metal following an exposure to luminous radiation, characterized in that the said agent contains a group containing at least one chelating functional group for a transition metal, blocked by a photocleavable substituent and in that it additionally includes a cosmetically and/or dermatologically acceptable medium.

9. Composition according to Claim 8, characterized in that the chelating functional group is chosen from amine, carbonyl, nitrile, oxime, carboxylic, hydroxyl, hydroxamic, alkoxy, enolic, phenolic, phenoxy, hydrazide and sulphur-containing functional groups and combinations thereof.

10. Composition according to Claim 8 or 9, characterized in that the group is chosen from hydroxypyridinones, dicarboxylic amines, o-hydroxybenzylamines and hydroxamates.

11. Composition according to any one of Claims 8 to 10, characterized in that the photocleavable substituent is chosen from p-methoxyphenacyl, 2-nitrobenzyl, 2-nitrobenzyloxycarbonyl, 2-nitrophenylethylene glycol, benzyloxycarbonyl, 3,5-dimethoxybenzyloxycarbonyl, α,α-dimethyl-3,5-dimethoxybenzyloxycarbonyl, 3-nitrophenyl, 3-nitrophenoxy, 3,5-dinitrophenoxy, 3-nitrophenoxycarbonyl, phenacyl, 4-methoxyphenacyl, α-methylphenacyl, 3,5-dimethoxybenzoinyl and 2,4-dinitrobenzenesulphenyl groups.

12. Composition according to any one of Claims 8 to 11, characterized in that the photocleavable substituent is chosen from p-methoxyphenacyl and o-nitrobenzyl groups.

13. Composition according to any one of Claims 8 to 12, characterized in that the transition metal has an atomic number chosen from the range running from 21 to 30.

14. Composition according to any one of Claims 8 to 13, characterized in that the transition metal is chosen from the group consisting of iron and copper.

15. Composition for protecting the skin, the hair and/or the mucosa against the effects induced by luminous radiation and/or for preventing and/or combating cutaneous photoaging, characterized in that it consists of a composition according to any one of Claims 8 to 14.

16. Use of an agent capable of chelating a transition metal following an exposure to luminous radiation, the said agent containing a group containing at least one chelating functional group blocked by at least one photocleavable substituent in a cosmetic and/or dermatological composition for protecting the skin, the hair and/or the mucosae against the effects induced by luminous radiation and/or for preventing and/or combating cutaneous photoaging.

17. Use of the composition according to any one of Claims 8 to 14 for preparing a dermatological salve and/or ointment which are intended for protecting the skin, the hair and/or the mucosae against the effects induced by luminous radiation and/or for preventing and/or combating cutaneous photoaging.

18. Process for cosmetic treatment of the skin, of the hair and/or of the mucosa, consisting in applying to the skin, the hair and/or the mucosae, the composition according to any one of Claims 8 to 14.

## Patentansprüche

1. Verwendung einer Verbindung als Mittel, das zur Chelatisierung eines Übergangsmetalls nach der Einwirkung von Lichtstrahlung befähigt ist, die eine Gruppierung enthält, die mindestens eine ein Übergangsmetall chelatisierende funktionelle Gruppe aufweist, welche durch mindestens einen photochemisch abspaltbaren Substituenten blockiert ist.

2. Verwendung nach Anspruch 1, dadurch gekennzeichnet, daß die chelatisierende funktionelle Gruppe des Chelatbildners unter Amin-, Carbonyl-, Nitril-, Oxim-, Carboxy-, Hydroxy-, Hydroxam-, Alkoxy-, Enol-, Phenol-, Phenoxy-und Hydrazidgruppen sowie schwefelhaltigen Gruppen sowie Kombinationen dieser Gruppen ausgewählt wird.

3. Verwendung nach Anspruch 1 oder 2, dadurch gekennzeichnet, daß die Gruppierung des Chelatbildners unter Hydroxypyridinonen, Aminodicarbonsäuren, o-Hydroxybenzylaminen und Hydroxamaten ausgewählt wird.

4. Verwendung nach einem der Ansprüche 1 bis 3, dadurch gekennzeichnet, daß der photochemisch abspaltbare Substituent des Chelatbildners unter p-Methoxyphenacyl, 2-Nitrobenzyl, 2-Nitrobenzyloxycarbonyl, 2-Nitrophenylethylenglykol, Benzyloxycarbonyl, 3,5-Dimethoxybenzyloxycarbonyl, α,α-Dimethyl-3,5-dimethoxybenzyloxycarbonyl, 3-Nitrophenyl, 3-Nitrophenyloxy, 3,5-Dinitrophenyloxy, 3-Nitrophenyloxycarbonyl, Phenacyl, 4-Methoxyphenibyl, α-Methylphenacyl, 3,5-Dimethoxy-benzoinyl und 2,4-Dinitrobenzolsulfenyl ausgewählt wird.

5. Verwendung nach einem der Ansprüche 1 bis 4, dadurch gekennzeichnet, daß der photochemisch abspaltbare Substituent des Chelatbildners unter p-Methoxyphenacyl und o-Nitrobenzyl ausgewählt wird.

6. Verwendung nach einem der Ansprüche 1 bis 5, dadurch gekennzeichnet, daß das Übergangsmetall eine im Bereich von 21 bis 30 ausgewählte Ordnungszahl aufweist.

7. Verwendung nach einem der Ansprüche 1 bis 6, dadurch gekennzeichnet, daß das Übergangsmetall unter Eisen und Kupfer ausgewählt wird.

8. Zusammensetzung, die ein Mittel enthält, das zur Chelatisierung eines Übergangsmetalls nach der Einwirkung von Lichtstrahlung befähigt ist, dadurch gekennzeichnet, daß das Mittel eine Gruppierung enthält, die mindestens eine ein Übergangsmetall chelatisierende funktionelle Gruppe aufweist, welche durch mindestens einen photochemisch abspaltbaren Substituenten blockiert ist, und daß sie ferner ein kosmetisch und/oder dermatologisch akzeptables Medium enthält.

9. Zusammensetzung nach Anspruch 8, dadurch gekennzeichnet, daß die chelatisierende funktionelle Gruppe unter Amin-, Carbonyl-, Nitril-, Oxim-, Carboxy-, Hydroxy-, Hydroxam-, Alkoxy-, Enol-, Phenol-, Phenoxy- und Hydrazidgruppen und schwefelhaltigen Gruppen sowie Kombinationen dieser Gruppen ausgewählt ist.

10. Zusammensetzung nach Anspruch 8 oder 9, dadurch gekennzeichnet, daß die Gruppierung des Chelatbildners unter Hydroxypyridinonen, Aminodicarbonsäuren, o-Hydroxy-benzylaminen und Hydroxamaten ausgewählt ist.

11. Zusammensetzung nach einem der Ansprüche 8 bis 10, dadurch gekennzeichnet, daß der photochemisch abspaltbare Substituent unter p-Methoxyphenacyl, 2-Nitrobenzyl, 2-Nitrobenzyloxycarbonyl, 2-Nitrophenylethylen-glykol, Benzyloxycarbonyl, 3,5-Dimethoxybenzyloxycar-bonyl, α,α-Dimethyl-3,5-dimethoxybenzyloxycarbonyl, 3-Nitrophenyl, 3-Nitrophenyloxy, 3,5-Dinitrophenyloxy, 3-Nitrophenyloxycarbonyl, Phenacyl, 4-Methoxyphenacyl, α-Methylphenacyl, 3,5-Dimethoxybenzoinyl und 2,4-Dinitrobenzolsulfenyl ausgewählt ist.

12. Zusammensetzung nach einem der Ansprüche 8 bis 11, dadurch gekennzeichnet, daß der photochemisch abspaltbare Substituent unter p-Methoxyphenacyl und o-Nitrobenzyl ausgewählt ist.

13. Zusammensetzung nach einem der Ansprüche 8 bis 12, dadurch gekennzeichnet, daß das Übergangsmetall eine im Bereich von 21 bis 30 ausgewählte Ordnungszahl aufweist.

14. Zusammensetzung nach einem der Ansprüche 8 bis 13, dadurch gekennzeichnet, daß das Übergangsmetall unter Eisen und Kupfer ausgewählt ist.

15. Zusammensetzung, die die Haut, die Haare und/oder die Schleimhäute vor den durch Lichtstrahlung hervorgerufenen Folgen schützt und/oder der lichtbedingten Hautalterung vorbeugt und/oder sie bekämpft, dadurch gekennzeichnet, daß sie aus einer Zusammensetzung nach einem der Ansprüche 8 bis 14 besteht.

16. Verwendung eines Mittels, das zur Chelatisierung eines Übergangsmetalls nach der Einwirkung von Lichtstrahlung befähigt ist, wobei das Mittel eine Gruppierung enthält, die mindestens eine chelatisierende funktionelle Gruppe aufweist, welche durch mindestens einen photochemisch abspaltbaren Substituenten blockiert ist, in einer kosmetischen und/oder dermatologischen Zusammensetzung, die die Haut, die Haare und/oder die Schleimhäute vor den durch Lichtstrahlung hervorgerufenen Folgen schützt und/oder der lichtbedingten Hautalterung vorbeugt und/oder sie bekämpft.

17. Verwendung der Zusammensetzung nach einem der Ansprüche 8 bis 14 zur Herstellung einer dermatologischen Pomade und/oder Salbe, die dafür bestimmt ist, die Haut, die Haare und/oder die Schleimhäute vor den durch die Bestrahlung mit Licht hervorgerufenen Folgen zu schützen und/oder der lichtbedingten Hautalterung vorzubeugen und/oder sie zu bekämpfen.

18. Verfahren zur kosmetischen Behandlung der Haut, der Haare und/oder der Schleimhäute, das darin besteht, auf die Haut, die Haare und/oder die Schleimhäute die Zusammensetzung nach einem der Ansprüche 8 bis 14 aufzutragen.
